# EUROPEAN PATENT APPLICATION

(11) **EP 4 342 886 A1**
(43) Date of publication of application: **27.03.2024**
(21) Application number: 22804627.2
(22) Date of filing: 16.05.2022
(51) Int. Cl.: C07D 319/12, C07D 317/32

(54) **METHOD FOR PRODUCING 1,4-DIOXANE DERIVATIVE, 1,4-DIOXANE DERIVATIVE, AND COMPOSITION CONTAINING 1,4-DIOXANE DERIVATIVE, METHOD FOR PRODUCING (1,3-DIOXOLANE)-2-CARBOXYLIC ACID DERIVATIVE, (1,3-DIOXOLANE)-2-CARBOXYLIC ACID DERIVATIVE, AND COMPOSITION CONTAINING (1,3-DIOXOLANE)-2-CARBOXYLIC ACID DERIVATIVE**

(30) Priority: 17.05.2021 JP 2021083519
(71) Applicant: Tosoh Corporation, Yamaguchi 746-8501 (JP); Tosoh Finechem Corporation, Shunan-shi, Yamaguchi 746-0006 (JP); Sagami Chemical Research Institute, Ayase-shi Kanagawa 252-1193 (JP)
(72) Inventor: INOUE Munenori, Ayase-shi, Kanagawa 252-1193 (JP); UEJI Tatsuya, Ayase-shi, Kanagawa 252-1193 (JP); SESOKO Yusuke, Shunan-shi, Yamaguchi 746-0006 (JP); ADACHI Hiroaki, Shunan-shi, Yamaguchi 746-0006 (JP); INOUE Daisuke, Shunan-shi, Yamaguchi 746-0006 (JP)
(74) Representative: Novagraaf International SA
(86) International application number: PCT/JP2022/020326
(87) International publication number: WO 2022/244715

(57) **Abstract**

The manufacturing method of the 1,4-dioxane derivative represented by Formula (2) and/or Formula (10) includes reacting water, an inorganic base, and a fluoroalkene oxide represented by Formula (1). The manufacturing method of the (1,3-dioxolane)-2-carboxylic acid derivative includes manufacturing the 1,4-dioxane derivative represented by Formula (2) and/or Formula (10) by the above manufacturing method and a manufacturing method of the (1,3-dioxolane)-2-carboxylic acid derivative includes manufacturing the 1,4-dioxane derivative represented by Formula (2) and/or Formula (10) comprising ring reduction under heating. In Formula (1), R represents a fluorine atom or a fluoroalkyl group. In Formula (2) and in Formula (10), R represents a fluorine atom or a fluoroalkyl group, two R's present are the same as or different from each other, and M^{'+} represents a counter cation.

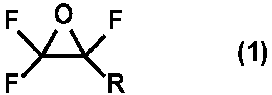

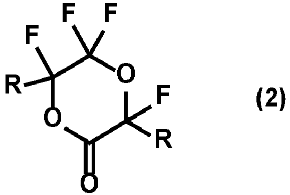

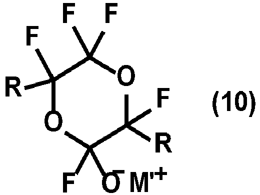

## Description

### [CROSS REFERENCE TO RELATED APPLICATIONS]

The present application claims priority from Japanese Patent Application No. 2021-083519, filed on May 17, 2021, the entire disclosure of which is particularly incorporated herein.

### [Technical field]

The present invention relates to a manufacturing method of a 1,4-dioxane derivative, a 1,4-dioxane derivative, a composition containing a 1,4-dioxane derivative, and a manufacturing method of a (1,3-dioxolane)-2-carboxylic acid derivative, a (1,3-dioxolane)-2-carboxylic acid derivative, and a composition containing a (1,3-dioxolane)-2-carboxylic acid derivative.

### [Background Art]

Poly[perfluoro(2-methylene-4-methyl-1,3-dioxolane)] can be obtained by polymerizing perfluoro(2-methylene-4-methyl-1,3-dioxolane) derived from a 2-[perfluoro(2,4-dimethyl-1,3-dioxolane)]carboxylic acid derivative. Poly[perfluoro(2-methylene-4-methyl-1,3-dioxolane)] is a promising polymer as a resin for gas separation membranes, a transparent resin for optical fibers, and the like.
(1,3-Dioxolane)-2-carboxylic acid derivatives such as 2-[perfluoro(2,4-dimethyl-1,3-dioxolane)]carboxylic acid derivatives are starting materials for the above promising polymer. As a manufacturing method of a 2-[perfluoro(2,4-dimethyl-1,3-dioxolane)]carboxylic acid derivative, PTL 1 and PTL 2 disclose the following method. Trifluoropyruvic acid fluoride prepared from hexafluoropropylene oxide and benzophenone is reacted with hexafluoropropylene oxide in a diethylene glycol dimethyl ether solvent in the presence of cesium fluoride to obtain perfluoro(dimethyl-2-oxo-1,4-dioxane). The obtained perfluoro(dimethyl-2-oxo-1,4-dioxane) is subjected to a ring contraction reaction under heating conditions in the presence of cesium fluoride to obtain 2-[perfluoro(2,4-dimethyl-1,3-dioxolane)]carboxylic acid fluoride.

Furthermore, PTL 2 discloses a method of obtaining the above perfluoro(dimethyl-2-oxo-1,4-dioxane) by causing a reaction with hexafluoropropylene oxide in a diethylene glycol dimethyl ether solvent in the presence of cesium fluoride using perfluoro(4-oxo-2,5-dimethyl-2-fluorocarbonyl-1,3-dioxane), obtained by reacting hexafluoropropylene oxide and benzophenone, as a starting material.

PTL 3 and PTL 4 disclose a method of obtaining perfluoro(dimethyl-2-oxo-1,4-dioxane) by reacting hexafluoropropylene oxide in the presence of dimethylformamide and hexamethylphosphoric acid triamide or in the presence of dimethylformamide and cesium fluoride. In addition, NPL 1 discloses a method of obtaining perfluoro(dimethyl-2-oxo-1,4-dioxane) by reacting hexafluoropropylene oxide with dimethylformamide.

[PTL 1] U.S. Patent No. 3308107
[PTL 2] U.S. Patent No. 3321517
[PTL 3] Japanese Patent Application Publication No. 1977-142078
[PTL 4] Japanese Patent No. 6766907

[NPL 1] Angew. Chem. Int. Ed. Engl. No. 24, pp. 161-179, 1985

### [Summary of Invention]

In the manufacturing method for obtaining perfluoro(dimethyl-2-oxo-1,4-dioxane) from hexafluoropropylene oxide, the methods disclosed in PTL 2, PTL 3, PTL 4, and NPL 1 require use of an organic carbonyl compound, such as benzophenone and dimethylformamide, as an oxygen source and a fluorine atom scavenger. However, because organic fluorine waste is generated as a by-product from these, separation and disposal thereof are required.

With the foregoing in view, an object of one aspect of the present invention is to provide a new manufacturing method of an intermediate for manufacturing a (1,3-dioxolane)-2-carboxylic acid derivative, a 1,4-dioxane derivative, a composition containing a 1,4-dioxane derivative, and a new manufacturing method of a (1,3-dioxolane)-2-carboxylic acid derivative, a (1,3-dioxolane)-2-carboxylic acid derivative, and a composition containing a (1,3-dioxolane)-2-carboxylic acid derivative.

As a result of extensive studies, the inventors of the present invention have newly found that a 1,4-dioxane derivative, which is an intermediate for manufacturing a (1,3-dioxolane)-2-carboxylic acid derivative, can be manufactured from water, an inorganic base, and a fluoroalkene oxide. That is, the inventors have found that a 1,4-dioxane derivative can be manufactured in a single step using water, which does not generate organic fluorine waste as a by-product, as an oxygen source, in place of an organic carbonyl compound that has been conventionally required. The inventors have also found that the obtained 1,4-dioxane derivative can be converted into a (1,3-dioxolane)-2-carboxylic acid derivative.

That is, one aspect of the present invention is as follows.
[1] A manufacturing method of one or more 1,4-dioxane derivatives selected from the group consisting of a 1,4-dioxane derivative represented by Formula (2) and a 1,4-dioxane derivative represented by Formula (10), the method including:
   reacting water, an inorganic base, and a fluoroalkene oxide represented by Formula (1), 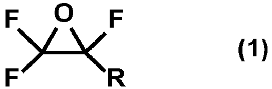 (in Formula (1), R represents a fluorine atom or a fluoroalkyl group), 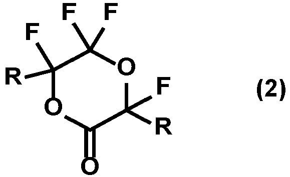 (in Formula (2), R represents a fluorine atom or a fluoroalkyl group, and two R's present are the same as or different from each other), and 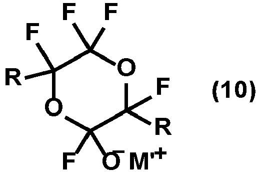 (in Formula (10), M^{'+} represents a counter cation, R represents a fluorine atom or a fluoroalkyl group, and two R's present are the same as or different from each other).
[2] The manufacturing method of a 1,4-dioxane derivative according to [1], in which the reaction is carried out in the presence of an organic solvent.
[3] The manufacturing method of a 1,4-dioxane derivative according to [2], in which the organic solvent is an ether solvent.
[4] The manufacturing method of a 1,4-dioxane derivative according to [3], in which the ether solvent is one or more ether solvents selected from the group consisting of ethylene glycol dimethyl ether, diethylene glycol dimethyl ether, triethylene glycol dimethyl ether, and tetraethylene glycol dimethyl ether.
[5] The manufacturing method of a 1,4-dioxane derivative according to [1], in which the inorganic base is one or more inorganic bases selected from the group consisting of alkali metal hydroxide salts, alkaline earth metal hydroxide salts, alkali metal carbonates, alkaline earth metal carbonates, alkali metal hydrogencarbonates, fluoride salts, and alkaline earth metal hydrogencarbonates.
[6] The manufacturing method of a 1,4-dioxane derivative according to [5], in which the inorganic base is one or more inorganic bases selected from the group consisting of alkali metal carbonates and fluoride salts.
[7] The manufacturing method of a 1,4-dioxane derivative according to [5], in which the inorganic base is an alkali metal carbonate.
[8] The manufacturing method of a 1,4-dioxane derivative according to [5], in which the inorganic base is a fluoride salt.
[9] The manufacturing method of a 1,4-dioxane derivative according to [1], in which a reaction temperature in the reaction is in a range of -20°C to 200°C.
[10] The manufacturing method of a 1,4-dioxane derivative according to [1], in which R is a fluoroalkyl group having 1 to 10 carbon atoms.
[11] The manufacturing method of a 1,4-dioxane derivative according to [1], in which R is a perfluoroalkyl group having 1 to 4 carbon atoms.
[12] A manufacturing method including:
   manufacturing a 1,4-dioxane derivative by the manufacturing method according to any one of [1] to [11] and
   the manufacturing method of one or more (1,3-dioxolane)-2-carboxylic acid derivatives selected from the group consisting of Formulas (5), (6), (7), and (9) including ring reduction of the manufactured 1,4-dioxane derivative under heating:
   a (1,3-dioxolane)-2-carboxylic acid fluoride represented by Formula (5): 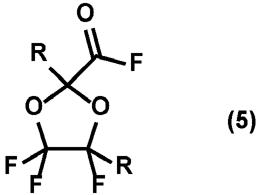 (in Formula (5), R represents a fluorine atom or a fluoroalkyl group, and two R's present are the same as or different from each other),
   a (1,3-dioxolane)-2-carboxylic acid represented by Formula (6): 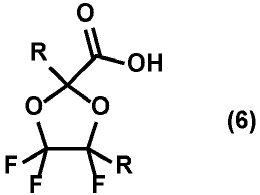 (in Formula (6), R represents a fluorine atom or a fluoroalkyl group, and two R's present are the same as or different from each other),
   a (1,3-dioxolane)-2-carboxylic acid salt represented by Formula (7): 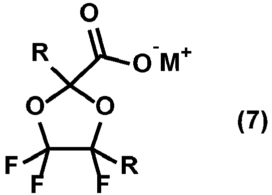 (in Formula (7), M⁺ represents a counter cation, R represents a fluorine atom or a fluoroalkyl group, and two R's present are the same as or different from each other), and
   a 1,3-dioxolane derivative represented by Formula (9): 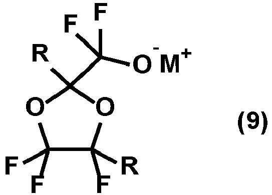 (in Formula (9), M⁺ represents a counter cation, R represents a fluorine atom or a fluoroalkyl group, and two R's present are the same as or different from each other).
[13] The manufacturing method of a (1,3-dioxolane)-2-carboxylic acid derivative according to [12], in which the ring contraction is performed in the presence of a fluoride.
[14] The manufacturing method of a (1,3-dioxolane)-2-carboxylic acid derivative according to [13], in which the fluoride is one or more fluorides selected from the group consisting of cesium fluoride, potassium fluoride, and sodium fluoride.
[15] The manufacturing method of a (1,3-dioxolane)-2-carboxylic acid derivative according to [12], in which a reaction temperature of the ring contraction is in a range of 100°C to 200°C.
[16] A 1,4-dioxane derivative represented by Formula (10), 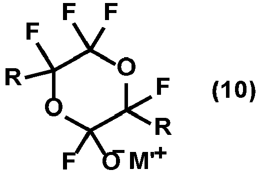 (in Formula (10), M^{'+} represents a counter cation, R represents a fluorine atom or a fluoroalkyl group, and two R's present are the same as or different from each other).
[17] A composition containing:
   a 1,4-dioxane derivative represented by Formula (2): 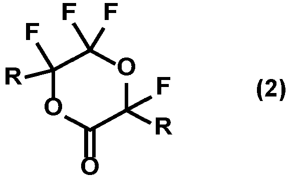 (in Formula (2), R represents a fluorine atom or a fluoroalkyl group in formula (1), and two R's present are the same as or different from each other); and
   a 1,4-dioxane derivative represented by Formula (10): 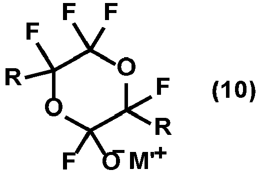 (in Formula (10), M^{'+} represents a counter cation, R represents a fluorine atom or a fluoroalkyl group, and two R's present are the same as or different from each other).
[18] A 1,3-dioxolane derivative represented by Formula (9): 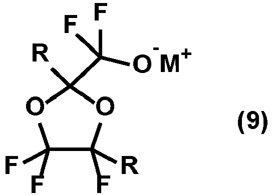 (in Formula (9), M⁺ represents a counter cation, R represents a fluorine atom or a fluoroalkyl group, and two R's are the same as or different from each other).
[19] A composition containing:
   one or more (1,3-dioxolane)-2-carboxylic acid derivatives selected from the group consisting of:
   a (1,3-dioxolane)-2-carboxylic acid fluoride represented by Formula (5): 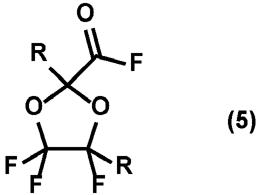 (in Formula (5), R represents a fluorine atom or a fluoroalkyl group, and two R's present are the same as or different from each other),
   a (1,3-dioxolane)-2-carboxylic acid represented by Formula (6): 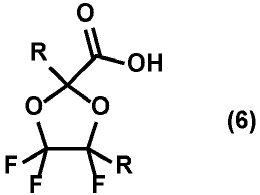 (in Formula (6), R represents a fluorine atom or a fluoroalkyl group, and two R's present are the same as or different from each other), and
   a (1,3-dioxolane)-2-carboxylic acid salt represented by Formula (7): 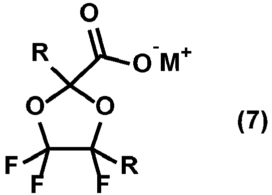 (in Formula (7), M⁺ represents a counter cation, R represents a fluorine atom or a fluoroalkyl group, and two R's present are the same as or different from each other); and
   a 1,3-dioxolane derivative represented by Formula (9): 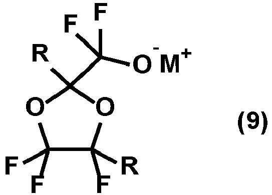 (in Formula (9), M⁺ represents a counter cation, R represents a fluorine atom or a fluoroalkyl group, and two R's present are the same as or different from each other).

According to one aspect of the present invention, it is possible to provide a new manufacturing method of a 1,4-dioxane derivative from water, an inorganic base, and a fluoroalkene oxide as starting materials, which are industrially inexpensive and readily available, by a single step. Such a manufacturing method is a manufacturing method not requiring an organic carbonyl compound that has been conventionally required.

Furthermore, the 1,4-dioxane derivative represented by Formula (10) obtained according to one aspect of the present invention can be converted to a (1,3-dioxolane)-2-carboxylic acid derivative by a low-pressure ring contraction reaction.

Furthermore, the 1,3-dioxolane derivative represented by Formula (9) obtained according to another aspect of the present invention can be converted to a (1,3-dioxolane)-2-carboxylic acid or a salt thereof by hydrolysis.

### [Description of Embodiments]

One aspect of the present invention relates to a manufacturing method of a 1,4-dioxane derivative. The above manufacturing method of a 1,4-dioxane derivative has a reaction step of reacting water, an inorganic base, and a fluoroalkene oxide to obtain a 1,4-dioxane derivative.

Another aspect of the present invention relates to a manufacturing method of a (1,3-dioxolane)-2-carboxylic acid derivative. The above manufacturing method of a (1,3-dioxolane)-2-carboxylic acid derivative involves reaction process for ring reduction converting the 1,4-dioxane derivative obtained by the method for manufacturing the 1,4-dioxane derivative to (1,3-dioxolane)-2.-carboxylic acid derivatives.

In the present invention and the present specification, the 1,4-dioxane derivative represents one or more compounds selected from the group consisting of a 1,4-dioxane derivative represented by Formula (2) above, and a 1,4-dioxane derivative represented by Formula (10) above. That is, the "1,4-dioxane derivative" in the present invention and the present specification includes the 1,4-dioxane derivative represented by Formula (2) and the 1,4-dioxane derivative represented by Formula (10). In one embodiment, a product of the above manufacturing method of a 1,4-dioxane derivative is one or more of the 1,4-dioxane derivative represented by Formula (2); one or more of the 1,4-dioxane derivative represented by Formula (10); or a mixture of one or more of the 1,4-dioxane derivative represented by Formula (2) and one or more of the 1,4-dioxane derivative represented by Formula (10).

In the present invention and the present specification, the (1,3-dioxolane)-2-carboxylic acid derivative represents one or more compounds selected from the group consisting of a (1,3-dioxolane)-2-carboxylic acid fluoride represented by Formula (5) above, a (1,3-dioxolane)-2-carboxylic acid represented by Formula (6) above, a (1,3-dioxolane)-2-carboxylic acid salt represented by Formula (7) above, and a 1,3-dioxolane derivative represented by Formula (9) above. That is, the "(1,3-dioxolane)-2-carboxylic acid derivative" in the present invention and the present specification includes the (1,3-dioxolane)-2-carboxylic acid represented by Formula (6), the (1,3-dioxolane)-2-carboxylic acid salt represented by Formula (7), the 1,3-dioxolane derivative represented by Formula (9), and the (1,3-dioxolane)-2-carboxylic acid fluoride represented by Formula (5). In one embodiment, a product of the above manufacturing method of a (1,3-dioxolane)-2-carboxylic acid derivative is one or more of the (1,3-dioxolane)-2-carboxylic acid fluoride represented by Formula (5); one or more of the (1,3-dioxolane)-2-carboxylic acid represented by Formula (6); one or more of the (1,3-dioxolane)-2-carboxylic acid salt represented by Formula (7); one or more of the 1,3-dioxolane derivative represented by Formula (9); a mixture of one or more of the (1,3-dioxolane)-2-carboxylic acid fluoride represented by Formula (5) and one or more of the (1,3-dioxolane)-2-carboxylic acid represented by Formula (6); a mixture of one or more of the (1,3-dioxolane)-2-carboxylic acid fluoride represented by Formula (5) and one or more of the 1,3-dioxolane derivative represented by Formula (9); a mixture of one or more of the 2-(1,3-dioxolane) carboxylic acid represented by Formula (6) and one or more of the 1,3-dioxolane derivative represented by Formula (9); a mixture of one or more of the (1,3-dioxolane)-2-carboxylic acid fluoride represented by Formula (5), one or more of the (1,3-dioxolane)-2-carboxylic acid represented by Formula (6), and one or more of the 1,3-dioxolane derivative represented by Formula (9); a mixture of one or more of the 2-(1,3-dioxolane) carboxylic acid salt represented by Formula (7) and one or more of the 1,3-dioxolane derivative represented by Formula (9); a mixture of one or more of the (1,3-dioxolane)-2-carboxylic acid fluoride represented by Formula (5), one or more of the (1,3-dioxolane)-2-carboxylic acid salt represented by Formula (7), and one or more of the 1,3-dioxolane derivative represented by Formula (9); a mixture of one or more of the 2-(1,3-dioxolane) carboxylic acid represented by Formula (6), one or more of the 2-(1,3-dioxolane) carboxylic acid salt represented by Formula (7), and one or more of the 1,3-dioxolane derivative represented by Formula (9); a mixture of one or more of the (1,3-dioxolane)-2-carboxylic acid fluoride represented by Formula (5), one or more of the (1,3-dioxolane)-2-carboxylic acid represented by Formula (6), one or more of the (1,3-dioxolane)-2-carboxylic acid salt represented by Formula (7), and one or more of the 1,3-dioxolane derivative represented by Formula (9); a mixture of one or more of the (1,3-dioxolane)-2-carboxylic acid represented by Formula (6) and one or more of the (1,3-dioxolane)-2-carboxylic acid salt represented by Formula (7); a mixture of one or more of the (1,3-dioxolane)-2-carboxylic acid fluoride represented by Formula (5) and one or more of the (1,3-dioxolane)-2-carboxylic acid salt represented by Formula (7); or a mixture of one or more of the (1,3-dioxolane)-2-carboxylic acid fluoride represented by Formula (5), one or more of the (1,3-dioxolane)-2-carboxylic acid represented by Formula (6), and one or more of the (1,3-dioxolane)-2-carboxylic acid salt represented by Formula (7).

Hereinbelow, the above manufacturing method of a 1,4-dioxane derivative, the above manufacturing method of a (1,3-dioxolane)-2-carboxylic acid derivative, and a composition will be described in more detail.

### [Manufacturing method of 1,4-dioxane derivative]

The above manufacturing method of a 1,4-dioxane derivative includes reacting water, an inorganic base, and a fluoroalkene oxide represented by Formula (1). Such a reaction is as shown in the reaction formula below. Details of R in the reaction formula below will be described later.

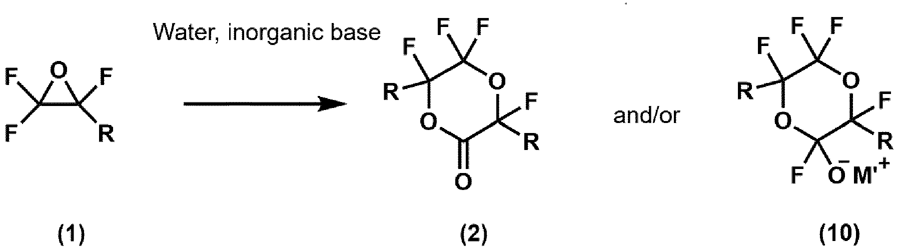

Hereinbelow, the fluoroalkene oxide represented by Formula (1) will be described.

In Formula (1), R represents a fluorine atom or a fluoroalkyl group.

In the present invention and the present specification, the "fluoroalkyl group" means a group in which one or more hydrogen atoms from an alkyl group are substituted with fluorine atoms, and also includes a perfluoroalkyl group in which all hydrogen atoms of an alkyl group are substituted with fluorine atoms.

Examples of the fluoroalkyl group represented by R include linear or branched fluoroalkyl groups such as a monofluoromethyl group, a difluoromethyl group, a trifluoromethyl group, a monofluoroethyl group, a difluoroethyl group, a trifluoroethyl group, a tetrafluoroethyl group, a pentafluoroethyl group, a monofluoropropyl group, a difluoropropyl group, a trifluoropropyl group, a tetrafluoropropyl group, a pentafluoropropyl group, a hexafluoropropyl group, a heptafluoropropyl group, a perfluorobutyl group, a perfluoropentyl group, a perfluorohexyl group, a perfluorooctyl group, a perfluorodecyl group, a perfluoroisopropyl group, and a perfluoro-t-butyl group. From the viewpoint of good yield, in the fluoroalkene oxide represented by Formula (1), R is preferably a fluoroalkyl group having 1 to 10 carbon atoms, more preferably a perfluoroalkyl group having 1 to 4 carbon atoms, and further preferably a trifluoromethyl group.

In the present invention and the present specification, R in each formula has the same definition as R in Formula (1), and the details thereof are as described above. In the formula, when two R's are present, these two R's are the same as or different from each other.

In Formula (10), M^{'+} represents a counter cation. The counter cation represented by M^{'+} can be a counter cation of the inorganic base used in the reaction or a counter cation of an inorganic salt added in a post-treatment. M^{'+} may be a single type of cation or multiple types of cations. Specific examples of monovalent counter cations include a cesium ion, a potassium ion, a sodium ion, a tetrabutylammonium ion, a tetraethylammonium ion, a tetrabutylphosphonium ion, a triethylmethylammonium ion, a triethylammonium ion, a pyridinium ion, and an ammonium ion. Examples of divalent counter cations include calcium ions and magnesium ions, and in the case of divalent counter cations, M^{'+} can be represented by (Ca²⁺)_{1/2}, (Mg²⁺)_{1/2}, or the like. From the viewpoint of availability and yield improvement, the counter cation represented by M^{'+} is preferably a cesium ion, a potassium ion, or a sodium ion, more preferably a cesium ion or a potassium ion, and further preferably a potassium ion.

Examples of the fluoroalkene oxide represented by Formula (1) include hexafluoropropylene oxide, perfluoro-1-butene oxide, perfluoro-1-pentene oxide, perfluoro-1-hexene oxide, perfluoro-1-heptene oxide, perfluoro-1-octene oxide, 2-(1,1,2,2-tetrafluoroethyl)-2,3,3-trifluorooxirane, 2-(1,1,2,2,3,3,4,4-octafluorobutyl)-2,3,3-trifluorooxirane, 2-(1,1,2,2,3,3,4,4,5,5,6,6-dodecafluorohexyl)-2,3,3-trifluorooxirane, 1,1,2,3-tetrafluoro-1-propylene oxide, and 1,1,2,3,3-pentafluoro-1-propylene oxide, among which hexafluoroalkene oxide is preferable. In the reaction, a single type of fluoroalkene oxide may be used, or a mixture of two or more types of fluoroalkene oxides at an arbitrary ratio may be used. For these fluoroalkene oxides, commercially available one can be used, or one prepared by oxidizing a corresponding fluoroalkene with an oxidant such as m-chloroperbenzoic acid can also be used.

The water provided for the above reaction is not particularly limited, and for example, tap water, ion-exchanged water, or distilled water can be used. In addition, water contained in an organic solvent to be described later and/or water contained in the inorganic base can also be the water provided for the reaction. The amount of water provided for the reaction can be a catalytic amount, for example, and is preferably an amount of 0.50 times or less by mol and more preferably an amount of 0.30 times or less by mol with respect to 1 mol of the fluoroalkene oxide represented by Formula (1). Furthermore, for example, the amount of water provided for the reaction can be an amount of 0.10 times or more by mol, an amount of 0.15 times or more by mol, an amount of 0.20 times or more by mol, or an amount of 0.21 times or more by mol with respect to 1 mol of the fluoroalkene oxide represented by Formula (1), or can be less than the values exemplified herein.

The inorganic base provided for the above reaction is not particularly limited. The inorganic base is preferably one or more inorganic bases selected from the group consisting of alkali metal hydroxide salts, alkaline earth metal hydroxide salts, alkali metal carbonates, alkaline earth metal carbonates, alkali metal hydrogencarbonates, fluoride salts, and alkaline earth metal hydrogencarbonates. Examples of such inorganic bases include alkali metal hydroxide salts such as sodium hydroxide, potassium hydroxide, and cesium hydroxide; alkaline earth metal hydroxide salts such as magnesium hydroxide and calcium hydroxide; alkali metal carbonates such as sodium carbonate, potassium carbonate, and cesium carbonate; alkaline earth metal carbonates such as magnesium carbonate and calcium carbonate; alkali metal hydrogencarbonates such as sodium hydrogencarbonate, potassium hydrogencarbonate, and cesium hydrogencarbonate; alkaline earth metal hydrogencarbonates such as magnesium hydrogencarbonate and calcium hydrogencarbonate; and fluoride salts such as cesium fluoride, potassium fluoride, sodium fluoride, potassium hydrogen fluoride, calcium fluoride, sodium hydrogen fluoride, tetrabutylammonium fluoride, tetraethylammonium fluoride, tetramethylammonium fluoride, tetrabutylphosphonium fluoride, and triethylmethylammonium fluoride. From the viewpoint of good yield, it is preferable to use one or more inorganic bases selected from the group consisting of alkali metal carbonates and fluoride salts as the inorganic base, and it is more preferable to use one or more inorganic bases selected from the group consisting of potassium carbonate and potassium fluoride. The amount of the inorganic base provided for the reaction is preferably an amount of 0.1 times or more by mol with respect to 1 mol of the fluoroalkene oxide represented by Formula (1). In addition, the amount of the inorganic base provided for the reaction is preferably an amount of 2 times or less by mol with respect to 1 mol of the fluoroalkene oxide represented by Formula (1). The molar ratio of the inorganic base to the water provided for the reaction is not particularly limited, but from the viewpoint of yield improvement, when using an alkali metal carbonate as the inorganic base, the molar ratio is preferably 0.90 or more and 1.25 or less, more preferably 0.95 or more and 1.20 or less, and further preferably 1.00 or more and 1.15 or less, and when using a fluoride salt as the inorganic base, the molar ratio is preferably 1.98 or more and 2.50 or less, more preferably 1.90 or more and 2.40 or less, and further preferably 2.00 or more and 2.50 or less.

The above reaction can be carried out in the presence or absence of an organic solvent, and is preferably carried out in the presence of an organic solvent from the viewpoint of good yield. In the above manufacturing method of a 1,4-dioxane derivative, for example, the 1,4-dioxane derivative represented by Formula (2) and/or Formula (10) can be obtained by charging an organic solvent, water, and the inorganic base in a pressure-resistant container, cooling, and thereafter adding the fluoroalkene oxide represented by Formula (1) thereto to cause a reaction.

The organic solvent is not particularly limited as long as it is inert to the reaction. Examples of the organic solvent include aromatic solvents such as toluene, ethylbenzene, xylene, and mesitylene; and ether solvents such as ethylene glycol dimethyl ether, diethylene glycol dimethyl ether (also called diglyme), triethylene glycol dimethyl ether, and tetraethylene glycol dimethyl ether. For the organic solvent, one type may be used alone, or two or more types may be mixed at an arbitrary ratio and used. As the organic solvent, it is preferable to use one type of ether solvents, such as ethylene glycol dimethyl ether, diethylene glycol dimethyl ether, triethylene glycol dimethyl ether, and tetraethylene glycol dimethyl ether, alone, or use two or more types thereof by mixing at an arbitrary ratio, and diethylene glycol dimethyl ether is particularly preferable. The amount of the organic solvent used is not particularly limited. For example, the organic solvent can be used in an amount of 0.3 part by weight or more with respect to 1 part by weight of the fluoroalkene oxide represented by Formula (1) provided for the reaction. In addition, the organic solvent can be used in an amount of 5.0 parts by weight or less with respect to 1 part by weight of the fluoroalkene oxide represented by Formula (1) provided for the reaction.

The above reaction can be carried out at a reaction temperature appropriately selected in the range of -20°C to 200°C, for example. The reaction temperature is preferably -20°C to 40°C from the viewpoint of good yield. The reaction temperature in the present invention and the present specification is a liquid temperature of a reaction liquid unless otherwise specified.

In one embodiment, a fluoride can be added to carry out the above reaction in the presence of one or more fluorides to accelerate the above reaction. Examples of fluorides include one or more fluorides selected from the group consisting of cesium fluoride, potassium fluoride, sodium fluoride, potassium hydrogen fluoride, calcium fluoride, sodium hydrogen fluoride, tetrabutylammonium fluoride, tetraethylammonium fluoride, tetramethylammonium fluoride, tetrabutylphosphonium fluoride, triethylmethylammonium fluoride, triethylamine trihydrofluoride, triethylamine pentahydrofluoride, triethylamine heptahydrofluoride, pyridinium poly(hydrogen fluoride), pyridine monohydrofluoride, pyridine nonahydrofluoride, ammonium hydrogen fluoride, and ammonium fluoride. Any of commercially available anhydrides, spray-dried products, and hydrates can be used as the fluoride. Alternatively, a form supported by calcium fluoride or the like may also be used. From the viewpoint of yield improvement, it is preferable to use one or more fluorides selected from the group consisting of cesium fluoride, potassium fluoride, and sodium fluoride, it is more preferable to use potassium fluoride, and it is further preferable to use an anhydride or spray-dried product of potassium fluoride. The amount of the fluoride added is not particularly limited. The amount of the fluoride is preferably an amount of 0.05 times or more by mol with respect to 1 mol of the fluoroalkene oxide (1) represented by Formula (1). The amount of the fluoride is preferably an amount of 1.0 times or less by mol with respect to 1 mol of the fluoroalkene oxide (1) represented by Formula (1).

If necessary, the 1,4-dioxane derivative represented by Formula (2) and/or Formula (10) obtained by the above reaction can be purified after the completion of the reaction. A purification method is not particularly limited. For example, the 1,4-dioxane derivative represented by Formula (2) and/or Formula (10) can be purified by methods, such as solvent extraction, silica gel column chromatography, preparative thin-layer chromatography, preparative liquid chromatography, and distillation, which are commonly used as purification methods by those skilled in the art.

In the present invention and the present specification, the 1,4-dioxane derivative represented by Formula (2) and/or Formula (10) includes the 1,4-dioxane derivative represented by Formula (2) and a 1,4-dioxane derivative represented by Formula (2a) as its diastereomer, and the 1,4-dioxane derivative represented by Formula (10) and a 1,4-dioxane derivative represented by Formula (10a) as its diastereomer. A product of the above manufacturing method of a 1,4-dioxane derivative can be only the 1,4-dioxane derivative represented by Formula (2), only the 1,4-dioxane derivative represented by Formula (2a), only the 1,4-dioxane derivative represented by Formula (10), only the 1,4-dioxane derivative represented by Formula (10a), or a mixture of two or more of the 1,4-dioxane derivative represented by Formula (2), the 1,4-dioxane derivative represented by Formula (2a), the 1,4-dioxane derivative represented by Formula (10), and the 1,4-dioxane derivative represented by Formula (10a). 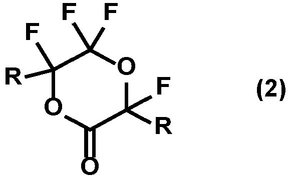 (in Formula (2), R represents a fluorine atom or a fluoroalkyl group, and two R's present are the same as or different from each other.) 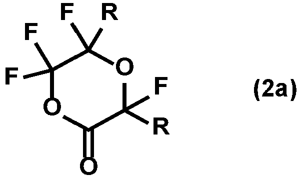 (in Formula (2a), R represents a fluorine atom or a fluoroalkyl group, and two R's present are the same as or different from each other.) 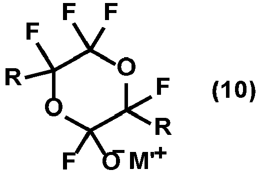 (in Formula (10), M^{'+} represents a counter cation, R represents a fluorine atom or a fluoroalkyl group, and two R's present are the same as or different from each other.) 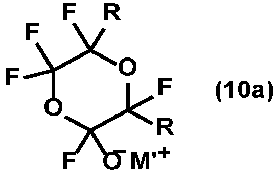 (in Formula (10a), M^{'+} represents a counter cation, R represents a fluorine atom or a fluoroalkyl group, and two R's present are the same as or different from each other.)

The 1,4-dioxane derivative represented by Formula (2) obtained by the above manufacturing method of a 1,4-dioxane derivative can be perfluoro(dimethyl-1,4-dioxane). Examples of the perfluoro(dimethyl-1,4-dioxane) include perfluoro(3,6-dimethyl-1,4-dioxane) and perfluoro(3,5-dimethyl-1,4-dioxane), and also include a mixture of these isomers.

The 1,4-dioxane derivative represented by Formula (10) obtained by the above manufacturing method of a 1,4-dioxane derivative can be a perfluoro(dimethyl-2-hydroxy-1,4-dioxane) potassium salt. Examples of the perfluoro(dimethyl-2-hydroxy-1,4-dioxane) potassium salt include a perfluoro(3,6-dimethyl-2-hydroxy-1,4-dioxane) potassium salt and a perfluoro(3,5-dimethyl -2-hydroxy-1,4-dioxane) potassium salt, and also include a mixture of these isomers.

### [Manufacturing method of (1,3-dioxolane)-2-carboxylic acid derivative]

Another aspect of the present invention relates to the manufacturing method of a (1,3-dioxolane)-2-carboxylic acid derivative.

For example, in the above manufacturing method of a (1,3-dioxolane)-2-carboxylic acid derivative, a (1,3-dioxolane)-2-carboxylic acid derivative can be obtained by providing, to a ring contraction step under heating, the 1,4-dioxane derivative, which is obtained in the above manufacturing method of a 1,4-dioxane derivative, in the form of the reaction mixture obtained in the above reaction as it is.

A product of the above manufacturing method of a (1,3-dioxolane)-2-carboxylic acid derivative is one or more (1,3-dioxolane)-2-carboxylic acid derivatives selected from the group consisting of the (1,3-dioxolane)-2-carboxylic acid fluoride represented by Formula (5), the (1,3-dioxolane)-2-carboxylic acid represented by Formula (6), the (1,3-dioxolane)-2-carboxylic acid salt represented by Formula (7), and the 1,3-dioxolane derivative represented by Formula (9).

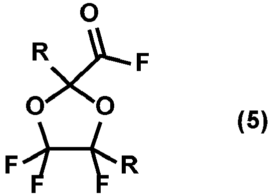

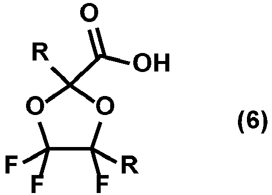

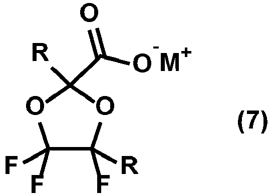

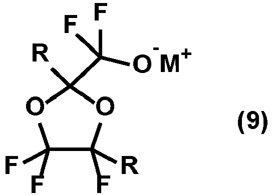

In the formulas above, R represents a fluorine atom or a fluoroalkyl group, and two R's present are the same as or different from each other. M⁺ in Formula (7) and M⁺ in Formula (9) represent a counter cation. The counter cation represented by M⁺ can be a counter cation of the inorganic base used in the reaction described above, a counter cation derived from the fluoride added in the ring contraction step, or a counter cation of an inorganic salt added in a post-treatment. M⁺ may be a single type of cation or multiple types of cations. Specific examples of monovalent counter cations include a cesium ion, a potassium ion, a sodium ion, a tetrabutylammonium ion, a tetraethylammonium ion, a tetrabutylphosphonium ion, a triethylmethylammonium ion, a triethylammonium ion, a pyridinium ion, and an ammonium ion. Examples of divalent counter cations include calcium ions and magnesium ions, and in the case of divalent counter cations, M⁺ can be represented by (Ca²⁺)_{1/2}, (Mg²⁺)_{1/2}, or the like. From the viewpoint of availability and yield improvement, the counter cation represented by M⁺ is preferably a cesium ion, a potassium ion, or a sodium ion, more preferably a cesium ion or a potassium ion, and further preferably a potassium ion.

The ring contraction step can be carried out by heating the reaction mixture obtained in the reaction described above to a reaction temperature appropriately selected from 100°C to 200°C, for example. Alternatively, heating may be performed under microwave irradiation, for example. The reaction temperature in the ring contraction step is preferably 100°C to 160°C from the viewpoint of good yield.

The reaction time for the ring contraction step is not particularly limited. For example, the ring contraction step can be completed by reacting for 1 hour to 72 hours. The ring contraction step is preferably carried out in a pressure-resistant container.

In one embodiment, the ring contraction step can be carried out in the presence of a fluoride to accelerate the reaction. Herein, the phrase "in the presence of" means the residue of the previous step or new addition. Examples of fluorides include one or more fluorides selected from the group consisting of cesium fluoride, potassium fluoride, sodium fluoride, potassium hydrogen fluoride, calcium fluoride, sodium hydrogen fluoride, tetrabutylammonium fluoride, tetraethylammonium fluoride, tetramethylammonium fluoride, tetrabutylphosphonium fluoride, triethylmethylammonium fluoride, triethylamine trihydrofluoride, triethylamine pentahydrofluoride, triethylamine heptahydrofluoride, pyridinium poly(hydrogen fluoride), pyridine monohydrofluoride, pyridine nonahydrofluoride, ammonium hydrogen fluoride, and ammonium fluoride. For example, the ring contraction step can be carried out by adding one or more fluorides to the reaction mixture containing the 1,4-dioxane derivative represented by Formula (2) and/or Formula (10). Any of commercially available anhydrides, spray-dried products, and hydrates can be used as the fluoride. Alternatively, a form supported by calcium fluoride or the like may also be used. From the viewpoint of yield improvement, it is preferable to use one or more fluorides selected from the group consisting of cesium fluoride, potassium fluoride, and sodium fluoride, it is more preferable to use potassium fluoride, and it is further preferable to use an anhydride or spray-dried product of potassium fluoride. The amount of the fluoride added is not particularly limited. For example, the amount of the fluoride added can be an amount of 0.05 times or more by mol with respect to 1 mol of the 1,4-dioxane derivative represented by Formula (2) and/or Formula (10). Furthermore, the amount of the fluoride added is preferably an amount of 1.0 times or less by mol with respect to 1 mol of the 1,4-dioxane derivative represented by Formula (2) and/or Formula (10), for example.

In addition, in one embodiment, an additive can be coexisted and reacted to improve the reactivity of the fluoride in the ring contraction step. By using the additive, the yield of the (1,3-dioxolane)-2-carboxylic acid derivative can be improved and/or the amount of the fluoride used can be reduced. Examples of such additives include crown ethers such as 18-crown-6 and 15-crown-5; polyetheramines such as tris[2-(2-methoxyethoxy)ethyl]amine (TDA-1); and polar solvents such as dimethylformamide and dimethyl sulfoxide. When the additive is a crown ether and/or polyetheramine, the amount of the additive used can be an amount of 0.05 times or more by mol, or can be an amount of 1.0 times or less by mol, for example, with respect to 1 mol of the 1,4-dioxane derivative represented by Formula (2) and/or Formula (10) which is provided to the ring contraction step.

In one embodiment, a pretreatment of the ring contraction step can be performed. As the pretreatment, for example, the following treatment can be performed. The reaction mixture before being subjected to the ring contraction step is heated to a temperature appropriately selected from a range of 40°C to 100°C, for example, and thereafter cooling to room temperature and depressurization are performed. The "room temperature" in the present invention and the present specification is a temperature in a range of 20°C to 25°C, for example.

In one embodiment, a (1,3-dioxolane)-2-carboxylic acid derivative as a desired product can be obtained by performing filtration and separation and removal of the upper layer of an organic solvent layer as a post-treatment after the ring contraction step, for example, after cooling to room temperature and depressurization. Alternatively, a desired product can be obtained by distilling the reaction mixture after the ring contraction step. Alternatively, a (1,3-dioxolane)-2-carboxylic acid derivative can be obtained by adding water or an alkaline aqueous solution such as an aqueous solution of potassium carbonate to the reaction mixture, and thereafter performing a separation and removal operation.

The (1,3-dioxolane)-2-carboxylic acid derivative obtained by the above manufacturing method can be a 2-[perfluoro(2,4-dimethyl-1,3-dioxolane)]carboxylic acid fluoride represented by Formula (5a) below and/or a perfluoro(2-hydroxymethyl-2,4-dimethyl-1,3-dioxolane) potassium salt represented by Formula (9a) below, for example. The 2-[perfluoro(2,4-dimethyl-1,3-dioxolane)]carboxylic acid fluoride represented by Formula (5a) below can lead to-a perfluoro(2-methylene-4-methyl-1,3-dioxolane) represented by Formula (8) below by the method disclosed in U.S. Patent No. 3308107 (PTL 1), for example.

In addition, the 2-[perfluoro(2,4-dimethyl-1,3-dioxolane)]carboxylic acid fluoride represented by Formula (5a) below and/or the perfluoro(2-hydroxymethyl-2,4-dimethyl-1,3-dioxolane) potassium salt represented by Formula (9a) below can be converted into the perfluoro(2-methylene-4-methyl-1,3-dioxolane) represented by Formula (8) below by a synthesis method shown in the following formula disclosed in Macromolecules 2005, vol. 38, pp. 4237-4245. That is, the perfluoro(2-methylene-4-methyl-1,3-dioxolane) represented by Formula (8) below can be manufactured by hydrolyzing the 2-[perfluoro(2,4-dimethyl-1,3-dioxolane)]carboxylic acid fluoride represented by Formula (5a) below and/or the perfluoro(2-hydroxymethyl-2,4-dimethyl-1,3-dioxolane) potassium salt represented by Formula (9a) below using an aqueous solution of potassium hydroxide to obtain a 2-[perfluoro(2,4-dimethyl-1,3-dioxolane)]carboxylic acid potassium salt represented by Formula (7a) below, and thereafter causing a decarboxylation reaction. The 2-[perfluoro(2,4-dimethyl-1,3-dioxolane)]carboxylic acid represented by Formula (6a) below or the potassium salt thereof, 2-[perfluoro(2,4-dimethyl-1,3-dioxolane)]carboxylic acid potassium salt represented by Formula (7a) below is an intermediate for synthesizing the perfluoro(2-methylene-4-methyl-1,3-dioxolane) represented by Formula (8) below from the 2-[perfluoro(2,4-dimethyl-1,3-dioxolane)]carboxylic acid fluoride represented by Formula (5a) below and/or the perfluoro(2-hydroxymethyl-2,4-dimethyl-1,3-dioxolane) potassium salt represented by Formula (9a) below. As described above, in the present invention and the present specification, the "(1,3-dioxolane)-2-carboxylic acid derivative" in the present invention and the present specification includes the (1,3-dioxolane)-2-carboxylic acid represented by Formula (6), the (1,3-dioxolane)-2-carboxylic acid salt represented by Formula (7), the 1,3-dioxolane derivative represented by Formula (9), and the (1,3-dioxolane)-2-carboxylic acid fluoride represented by Formula (5). Accordingly, the 2-[perfluoro(2,4-dimethyl-1,3-dioxolane)]carboxylic acid represented by Formula (6a) below and the 2-[perfluoro(2,4-dimethyl-1,3-dioxolane)]carboxylic acid potassium salt represented by Formula (7a) below are equivalent to the 2-[perfluoro(2,4-dimethyl-1,3-dioxolane)]carboxylic acid fluoride represented by Formula (5a) below and the perfluoro(2-hydroxymethyl-2,4-dimethyl-1,3-dioxolane) potassium salt represented by Formula (9a) below.

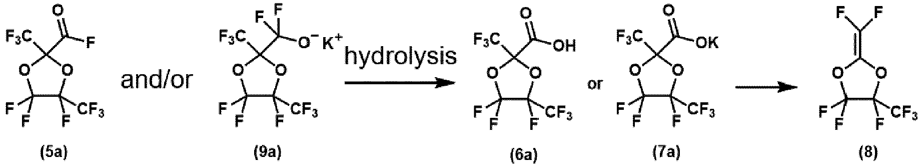

Examples of the (1,3-dioxolane)-2-carboxylic acid derivative represented by Formula (5) include 2-[perfluoro(2,4-dimethyl-1,3-dioxolane)]carboxylic acid fluoride, 2-[perfluoro(2,4-diethyl-1,3-dioxolane)]carboxylic acid fluoride, 2-[perfluoro(2,4-di-n-propyl-1,3-dioxolane)]carboxylic acid fluoride, 2-[perfluoro(2,4-di-n-butyl-1,3-dioxolane)]carboxylic acid fluoride, 2-[perfluoro(2,4-di-n-pentyl-1,3-dioxolane)]carboxylic acid fluoride, 2-[perfluoro(2,4-di-n-hexyl-1,3-dioxolane)]carboxylic acid fluoride, 2-[2,4-bis(1,1,2,2-tetrafluoroethyl)-4,5,5-trifluoro-1,3-dioxolane]carboxylic acid fluoride, 2-[2,4-bis(1,1,2,2,3,3,4,4,-octafluorobutyl)-4,5,5-trifluoro-1,3-dioxolane]carboxylic acid fluoride, 2-[2,4-bis(1,1,2,2,3,3,4,4,5,5,6,6-dodecafluorohexyl)-4,5,5-trifluoro-1,3 -dioxolane]carboxylic acid fluoride and the like.

Examples of the (1,3-dioxolane)-2-carboxylic acid derivative represented by Formula (6) include 2-[perfluoro(2,4-dimethyl-1,3-dioxolane)]carboxylic acid, 2-[perfluoro(2,4-diethyl-1,3-dioxolane)]carboxylic acid, 2-[perfluoro(2,4-di-n-propyl-1,3-dioxolane)]carboxylic acid, 2-[perfluoro(2,4-di-n-butyl-1,3-dioxolane)]carboxylic acid, 2-[perfluoro(2,4-di-n-pentyl-1,3-dioxolane)]carboxylic acid, 2-[perfluoro(2,4-di-n-hexyl-1,3-dioxolane)]carboxylic acid, 2-[2,4-bis(1,1,2,2-tetrafluoroethyl)-4,5,5-trifluoro-1,3-dioxolane]carboxylic acid, 2-[2,4-bis(1,1,2,2,3,3,4,4,-octafluorobutyl)-4,5,5-trifluoro-1,3-dioxolane]carboxylic acid, 2-[2,4-bis(1,1,2,2,3,3,4,4,5,5,6,6-dodecafluorohexyl)-4,5,5-trifluoro-1,3 -dioxolane]carboxylic acid and the like.

Examples of the (1,3-dioxolane)-2-carboxylic acid derivative represented by Formula (7) include 2-[perfluoro(2,4-dimethyl-1,3-dioxolane)]carboxylic acid potassium salt, 2-[perfluoro(2,4-diethyl-1,3-dioxolane)]carboxylic acid potassium salt, 2-[perfluoro(2,4-di-n-propyl-1,3-dioxolane)]carboxylic acid potassium salt, 2-[perfluoro(2,4-di-n-butyl-1,3-dioxolane)]carboxylic acid potassium salt, 2-[perfluoro(2,4-di-n-pentyl-1,3-dioxolane)]carboxylic acid potassium salt, 2-[perfluoro(2,4-di-n-hexyl-1,3-dioxolane)]carboxylic acid potassium salt, 2-[2,4-bis(1,1,2,2-tetrafluoroethyl)-4,5,5-trifluoro-1,3-dioxolane]carboxylic acid potassium salt, 2-[2,4-bis(1,1,2,2,3,3,4,4,-octafluorobutyl)-4,5,5-trifluoro-1,3-dioxolane]carboxylic acid potassium salt, 2-[2,4-bis(1,1,2,2,3,3,4,4,5,5,6,6-dodecafluorohexyl)-4,5,5-trifluoro-1,3 -dioxolane]carboxylic acid potassium salt, 2-[perfluoro(2,4-dimethyl-1,3-dioxolane)]carboxylic acid sodium salt, 2-[perfluoro(2,4-diethyl-1,3-dioxolane)]carboxylic acid sodium salt, 2-[perfluoro(2,4-di-n-propyl-1,3-dioxolane)]carboxylic acid sodium salt, 2-[perfluoro(2,4-di-n-butyl-1,3-dioxolane)]carboxylic acid sodium salt, 2-[perfluoro(2,4-di-n-pentyl-1,3-dioxolane)]carboxylic acid sodium salt, 2-[perfluoro(2,4-di-n-hexyl-1,3-dioxolane)]carboxylic acid sodium salt, 2-[2,4-bis(1,1,2,2-tetrafluoroethyl)-4,5,5-trifluoro-1,3-dioxolane]carboxylic acid sodium salt, 2-[2,4-bis(1,1,2,2,3,3,4,4,-octafluorobutyl)-4,5,5-trifluoro-1,3-dioxolane]carboxylic acid sodium salt, 2-[2,4-bis(1,1,2,2,3,3,4,4,5,5,6,6-dodecafluorohexyl)-4,5,5-trifluoro-1,3 -dioxolane]carboxylic acid sodium salt and the like.

Examples of the (1,3-dioxolane)-2-carboxylic acid derivative represented by Formula (9) include a perfluoro(2-hydroxymethyl-2,4-dimethyl-1,3-dioxolane) potassium salt, a perfluoro(2-hydroxymethyl-2,4-diethyl-1,3-dioxolane) potassium salt, a perfluoro(2-hydroxymethyl-2,4-di-n-propyl-1,3-dioxolane) potassium salt, a perfluoro(2-hydroxymethyl-2,4-di-n-butyl-1,3-dioxolane) potassium salt, a perfluoro(2-hydroxymethyl-2,4-di-n-pentyl-1,3-dioxolane) potassium salt, a perfluoro(2-hydroxymethyl-2,4-di-n-hexyl-1,3-dioxolane) potassium salt, a 2-(difluorohydroxymethyl)-2,4-bis(1,1,2,2-tetrafluoroethyl)-4,5,5-trifluoro-1,3-dioxolane potassium salt, a 2-(difluorohydroxymethyl)-2,4-bis(1,1,2,2,3,3,4,4,-octafluorobutyl)-4,5,5-trifluoro-1,3-dioxolane potassium salt, a 2-(difluorohydroxymethyl)-2,4-bis(1,1,2,2,3,3,4,4,5,5,6,6-dodecafluorohexyl)-4,5,5-trifluoro-1,3-dioxolane potassium salt, a perfluoro(2-hydroxymethyl-2,4-dimethyl-1,3-dioxolane) sodium salt, a perfluoro(2-hydroxymethyl-2,4-diethyl-1,3-dioxolane) sodium salt, a perfluoro(2-hydroxymethyl-2,4-di-n-propyl-1,3-dioxolane) sodium salt, a perfluoro(2-hydroxymethyl-2,4-di-n-butyl-1,3-dioxolane) sodium salt, a perfluoro(2-hydroxymethyl-2,4-di-n-pentyl-1,3-dioxolane) sodium salt, a perfluoro(2-hydroxymethyl-2,4-di-n-hexyl-1,3-dioxolane) sodium salt, a 2-(difluorohydroxymethyl)-2,4-bis(1,1,2,2-tetrafluoroethyl)-4,5,5-trifluoro-1,3-dioxolane sodium salt, a 2-(difluorohydroxymethyl)-2,4-bis(1,1,2,2,3,3,4,4,-octafluorobutyl)-4,5,5-trifluoro-1,3-dioxolane sodium salt, a 2-(difluorohydroxymethyl)-2,4-bis(1,1,2,2,3,3,4,4,5,5,6,6-dodecafluorohexyl)-4,5,5-trifluoro-1,3-dioxolane sodium salt and the like.

### [Composition]

Still another aspect of the present invention relates to a composition (composition A) containing the 1,4-dioxane derivative represented by Formula (2) and the 1,4-dioxane derivative represented by Formula (10).

In addition, still another aspect of the present invention relates to a composition (composition B) containing one or more (1,3-dioxolane)-2-carboxylic acid derivatives selected from the group consisting of the (1,3-dioxolane)-2-carboxylic acid fluoride represented by Formula (5), the (1,3-dioxolane)-2-carboxylic acid represented by Formula (6), and the (1,3-dioxolane)-2-carboxylic acid salt represented by Formula (7), and the 1,3-dioxolane derivative represented by Formula (9).

In the composition A, the content ratio of the 1,4-dioxane derivative represented by Formula (2) and the 1,4-dioxane derivative represented by Formula (10) is preferably perfluoro(dimethyl-2-oxo-1,4-dioxane):perfluoro(dimethyl-2-hydroxy-1,4-dioxane) potassium salt = 1:0.01 to 1:1, and more preferably 1:0.02 to 1:0.20.

In the composition B, the content ratio of one or more (1,3-dioxolane)-2-carboxylic acid derivatives selected from the group consisting of the (1,3-dioxolane)-2-carboxylic acid fluoride represented by Formula (5), the (1,3-dioxolane)-2-carboxylic acid represented by Formula (6), and the (1,3-dioxolane)-2-carboxylic acid salt represented by Formula (7), and the 1,3-dioxolane derivative represented by Formula (9) is preferably one or more (1,3-dioxolane)-2-carboxylic acid derivatives selected from the group consisting of the (1,3-dioxolane)-2-carboxylic acid fluoride represented by Formula (5), the (1,3-dioxolane)-2-carboxylic acid represented by Formula (6), and the (1,3-dioxolane)-2-carboxylic acid salt represented by Formula (7):1,3-dioxolane derivative represented by Formula (9) = 0.01:1 to 100:1, and more preferably 0.02:1 to 50:1.

### [Examples]

Hereinbelow, the present invention will be further described with reference to Examples. However, the present invention is not limited to the embodiments described in the Examples.

The following instruments were used in the analysis below.

¹⁹F NMR: AVANCE II 400 manufactured by Bruker Corporation or GSX-400 spectrometer (JEOL GSX-400 spectrometer) manufactured by JEOL Ltd.

### [Example 1]

Diethylene glycol dimethyl ether (9.37 g, 69.8 mmol), potassium carbonate (1.10 g, 7.90 mmol), and water (0.14 g, 7.8 mmol) were charged into a 30 mL autoclave made of SUS316 under an argon atmosphere. After cooling to -78°C with a dry ice/acetone bath, hexafluoropropylene oxide (6.60 g, 39.8 mmol) was introduced and the mixture was stirred at 0°C (reaction temperature) for 18 hours. When depressurization was carried out to distill the obtained solution under reduced pressure at 50°C, 4.28 g of a fraction was obtained. When NMR analysis of this fraction was performed with ¹⁹F NMR using hexafluorobenzene as an internal standard substance, it was confirmed that 3.83 g (yield 62%/hexafluoropropylene oxide basis) of a perfluoro(dimethyl-2-oxo-1,4-dioxane) (Formula (2)) was generated. It was confirmed that a perfluoro(dimethyl-2-oxo-1,4-dioxane) was generated as two types of diastereomers.

¹⁹F NMR(376MHz,CDCl₃) (isomer 1) δ(ppm):-81.6,-81.8,-82.2,-83.0,-117.2,-126.3_{∘} (isomer 2) δ(ppm):-81.6,-82.2,-83.0,-94.6,-113.7,-128.5.

### [Example 2]

Diethylene glycol dimethyl ether (9.37 g, 69.8 mmol), potassium carbonate (1.09 g, 7.89 mmol), and water (0.14 g, 7.8 mmol) were charged into a 30 mL autoclave made of SUS316 under an argon atmosphere. After cooling to -78°C with a dry ice/acetone bath, hexafluoropropylene oxide (6.60 g, 39.8 mmol) was introduced and the mixture was stirred at 0°C (reaction temperature) for 18 hours.

Subsequently, potassium fluoride (0.58 g, 10.0 mmol) and diethylene glycol dimethyl ether (2.81 g, 21.0 mmol) were added to the reaction mixture obtained above under an argon atmosphere. As a pretreatment of the ring contraction step, the mixture was heated at 80°C for 1 hour, cooled to room temperature, depressurized, and thereafter replaced with argon.

Thereafter, the temperature was raised to 130°C, and the reaction (ring contraction step) was carried out for 18 hours.

When the obtained solution was distilled under reduced pressure at 50°C, 2.82 g of a fraction was obtained. When NMR analysis of this fraction was performed with ¹⁹F NMR using hexafluorobenzene as an internal standard substance, it was confirmed that 2.65 g (yield 43%/hexafluoropropylene oxide basis) of a 2-[perfluoro(2,4-dimethyl-1,3-dioxolane)]carboxylic acid fluoride (Formula (5a)) was generated. In addition, when 30 mL of water was added to the residue after distillation under reduced pressure, and potassium carbonate was added to a part of the collected solution to perform NMR analysis with ¹⁹F NMR with trifluoroacetic acid as an internal standard substance, it was confirmed that 1.72 g (yield 25%/hexafluoropropylene oxide basis) of a 2-[perfluoro(2,4-dimethyl-1,3-dioxolane)]carboxylic acid potassium salt (Formula (7a)) was generated (total yield 68% of a 2-[perfluoro(2,4-dimethyl-1,3-dioxolane)]carboxylic acid derivative).

It was confirmed that the obtained 2-[perfluoro(2,4-dimethyl-1,3-dioxolane)]carboxylic acid fluoride was generated as two types of diastereomers.

¹⁹F NMR(376MHz,CDCl₃) (isomer 1) δ(ppm):23.6,-77.8(d,J=132Hz),-80.1,-81.57,-83.56(d,J=135Hz),-124.9_{∘} (isomer 2) δ(ppm):23.2,-78.5(d,J=132Hz),-80.4,-81.6,-84.1(d,J=139Hz),-123.7.

In addition, it was confirmed that the 2-[perfluoro(2,4-dimethyl-1,3-dioxolane)]carboxylic acid potassium salt was also generated as two types of diastereomers.

¹⁹F NMR(376MHz,D₂O) (isomer 1) δ(ppm):-78.8,-81.0,-81.9,-84.4,-124.8_{∘} (isomer 2) δ(ppm):-79.5,-81.4,-82.0,-84.8,-124.9.

### [Example 3]

Diethylene glycol dimethyl ether (9.37 g, 69.8 mmol), potassium carbonate (1.08 g, 7.8 mmol), and water (0.14 g, 7.8 mmol) were charged into a 30 mL autoclave made of SUS316 under an argon atmosphere. After cooling to -78°C with a dry ice/acetone bath, hexafluoropropylene oxide (5.50 g, 33.1 mmol) was introduced and the mixture was stirred at 0°C (reaction temperature) for 18 hours. When depressurization was carried out to distill the obtained solution under reduced pressure at 50°C, 4.67 g of a fraction was obtained. When NMR analysis of this fraction was performed with ¹⁹F NMR using hexafluorobenzene as an internal standard substance, it was confirmed that 4.51 g (yield 88%/hexafluoropropylene oxide basis) of a perfluoro(dimethyl-2-oxo-1,4-dioxane) (Formula (2)) was generated. It was confirmed that a perfluoro(dimethyl-2-oxo-1,4-dioxane) was generated as two types of diastereomers.

### [Example 4]

Diethylene glycol dimethyl ether (34.67 g), potassium carbonate (4.623 g, 33.45 mmol), and water (0.622 g, 34.52 mmol) were charged into a 100 mL autoclave made of SUS316 under a nitrogen atmosphere. After cooling to 0°C with an ice bath, hexafluoropropylene oxide (21.71 g, 130.77 mmol) was introduced and the mixture was stirred at 0°C (reaction temperature) for 18 hours. After stirring at 20°C to 25°C for 2 hours, depressurization was performed after cooling to 0°C again, and the reaction liquid was taken out from the container. When NMR analysis of this reaction liquid was performed with ¹⁹F NMR using benzotrifluoride as an internal standard substance, it was confirmed that 11.16 g (yield 55%/hexafluoropropylene oxide basis) of a perfluoro(dimethyl-2-oxo-1,4-dioxane) (Formula (2)) was generated.

### [Examples 5 to 8]

A perfluoro(dimethyl-2-oxo-1,4-dioxane) (Formula (2)) and a perfluoro(dimethyl-2-hydroxy-1,4-dioxane) potassium salt (Formula (10)) were obtained by performing the same operation as in Example 4 except that the size of a reaction container, and the charging amounts of diethylene glycol dimethyl ether, potassium carbonate, water, and hexafluoropropylene oxide were changed as shown in Table 1. Table 1 shows the results.

Examples 6 to 8 were compositions containing a perfluoro(dimethyl-2-oxo-1,4-dioxane) and a perfluoro(dimethyl-2-hydroxy-1,4-dioxane) potassium salt, and the content ratio was perfluoro(dimethyl-2-oxo-1,4-dioxane):perfluoro(dimethyl-2-hydroxy-1,4-dioxane) potassium salt = 1:0.09 (Example 6), 1:0.18 (Example 7), and 1:0.25 (Example 8).

**[Table 1]**

| Example | | Example 5 | Example 6 | Example 7 | Example 8 |
|---|---|---|---|---|---|
| Size of reaction container | | 190mL | 190mL | 100mL | 100mL |
| Charging amount | Diethylene glycol dimethyl ether | 59.22g | 60.77g | 33.91g | 31.74g |
| | Potassium carbonate | 8.306g (60.10mmol) | 8.781g (63.53mmol) | 4.622g (33.45mmol) | 4.615g (33.39mmol) |
| | Water | 1.072g (59.53mmol) | 1.071g (59.47mmol) | 0.533g (29.57mmol) | 0.514g (28.54mmol) |
| | Hexafluoropropylene oxide | 41.11g (247.62mmol) | 40.98g (246.84mmol) | 21.43g (129.08mmol) | 21.40g (128.90mmol) |
| Yield | Perfluoro(dimethyl-2-oxo-1,4-dioxane) | 63% | 59% | 54% | 47% |
| | Perfluoro(dimethyl-2-hydroxy-1,4-dioxane) potassium salt | 0% | 5% | 10% | 12% |

The analysis results of the perfluoro(dimethyl-2-hydroxy-1,4-dioxane) potassium salt were as follows.

¹⁹F NMR (376 MHz, no solvent) δ(ppm):-24.63,-74.97(d,J=150Hz),-81.72,-82.58,-86.25,-122.34. -129.98.

### [Example 9]

Diethylene glycol dimethyl ether (77.87 g), potassium carbonate (11.192 g, 80.98 mmol), potassium fluoride (4.641 g, 79.88 mmol, spray-dried), and water (1.443 g, 80.12 mmol) were charged into a 250 mL autoclave made of SUS316 under a nitrogen atmosphere. After cooling to 0°C with an ice bath, hexafluoropropylene oxide (55.20 g, 332.49 mmol) was introduced and the mixture was stirred at 0°C (reaction temperature) for 18 hours. After stirring at 20°C to 25°C for 2 hours, depressurization was performed after cooling to 0°C again, and the reaction liquid was taken out from the container. When NMR analysis of this reaction liquid was performed with ¹⁹F NMR using benzotrifluoride as an internal standard substance, it was confirmed that 24.91 g (yield 41%/hexafluoropropylene oxide basis) of a perfluoro(dimethyl-2-hydroxy-1,4-dioxane) potassium salt (Formula (10)) was generated.

### [Example 10]

Diethylene glycol dimethyl ether (59.37 g), potassium fluoride (14.244 g, 245.18 mmol, spray-dried), and water (2.101 g, 116.65 mmol) were charged into a 190 mL autoclave made of SUS316 under a nitrogen atmosphere. After cooling to 0°C with an ice bath, hexafluoropropylene oxide (41.10 g, 247.56 mmol) was introduced and the mixture was stirred at 0°C (reaction temperature) for 66 hours. After stirring at 20°C to 25°C for 2 hours, depressurization was performed after cooling to 0°C again, and the reaction liquid was taken out from the container. When NMR analysis of this reaction liquid was performed with ¹⁹F NMR using benzotrifluoride as an internal standard substance, it was confirmed that 20.42 g (yield 53%/hexafluoropropene oxide basis) of a perfluoro(dimethyl-2-hydroxy-1,4-dioxane) potassium salt (Formula (10)) was generated.

### [Example 11]

16.13 g of the reaction liquid obtained in Example 5 (containing 3.72 g (12.00 mmol) of the perfluoro(dimethyl-2-oxo-1,4-dioxane)), diethylene glycol dimethyl ether (2.79 g), and potassium fluoride (0.556 g, 9.58 mmol, spray-dried) were charged into a 30 mL autoclave made of SUS316 under a nitrogen atmosphere. As a pretreatment of the ring contraction step, the mixture was heated at 80°C for 1 hour, cooled to 0°C, and depressurized.

Thereafter, the temperature was raised to 120°C, and the reaction (ring contraction step) was carried out for 24 hours.

When NMR analysis of the obtained solution was performed with ¹⁹F NMR using benzotrifluoride as an internal standard substance, it was confirmed that 3.26 g (yield 74%/perfluoro(dimethyl-2-oxo-1,4-dioxane) basis) of a perfluoro(2-hydroxymethyl-2,4-dimethyl-1,3-dioxolane) potassium salt (Formula (9a)) was generated.

The analysis results of the perfluoro(2-hydroxymethyl-2,4-dimethyl-1,3-dioxolane) potassium salt were as follows.

¹⁹F NMR (376 MHz, no solvent) δ(ppm):-31.62,-77.54(d,J=132Hz),-80.11,-81.11,-81.66,-82.00~-83.50,-121.83.

### [Example 12]

21.73 g of the reaction liquid obtained in Example 9 (containing 3.74 g (10.15 mmol) of the perfluoro(dimethyl-2-hydroxy-1,4-dioxane) potassium salt (Formula (10))) was charged into a 30 mL autoclave made of SUS316 under a nitrogen atmosphere. The temperature was raised to 130°C, and the reaction (ring contraction step) was carried out for 66 hours. In the reaction liquid obtained in Example 9, diethylene glycol dimethyl ether was present.

When NMR analysis of the obtained solution was performed with ¹⁹F NMR using benzotrifluoride as an internal standard substance, it was confirmed that 3.48 g (yield 93%/perfluoro(dimethyl-2-hydroxy-1,4-dioxane) potassium salt basis) of a perfluoro(2-hydroxymethyl-2,4-dimethyl-1,3-dioxolane) potassium salt and 0.10 g (yield 3%/perfluoro(dimethyl-2-hydroxy-1,4-dioxane) potassium salt basis) of a [perfluoro(2,4-dimethyl-1,3-dioxolane)]carboxylic acid fluoride were generated.

### [Example 13]

An aqueous solution (7.48 g) of 25% potassium hydroxide was charged into a 30 mL eggplant flask and the mixture was cooled to 0°C in an ice bath, and while stirring with a stirring bar, 11.72 g of the reaction liquid obtained in Example 11 (containing 2.15 g (5.85 mmol) of the perfluoro(2-hydroxymethyl-2,4-dimethyl-1,3-dioxolane) potassium salt) was added dropwise. The reaction liquid separated into two layers. When NMR analysis of the upper layer was performed with ¹⁹F NMR using 2,2,2-trifluoroethanol as an internal standard substance, it was confirmed that 2.02 g (yield 100%/perfluoro(2-hydroxymethyl-2,4-dimethyl-1,3-dioxolane) potassium salt basis) of a 2-[perfluoro(2,4-dimethyl-1,3-dioxolane)]carboxylic acid potassium salt was generated.

The (1,3-dioxolane)-2-carboxylic acid derivative obtained according to one aspect of the present invention can be used as a synthetic starting material for poly[perfluoro(2-methylene-4-methyl-1,3-dioxolane)] which is promising as a resin for gas separation membranes, a transparent resin for optical fibers, and the like.

## Claims

1. A manufacturing method of one or more 1,4-dioxane derivatives selected from the group consisting of a 1,4-dioxane derivative represented by Formula (2) and a 1,4-dioxane derivative represented by Formula (10), the method comprising:
reacting water, an inorganic base, and a fluoroalkene oxide represented by Formula (1), 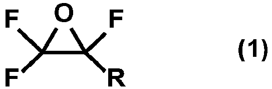 (in Formula (1), R represents a fluorine atom or a fluoroalkyl group), 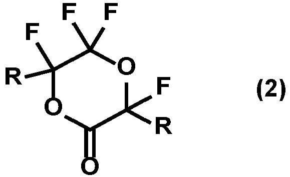 (in Formula (2), R represents a fluorine atom or a fluoroalkyl group, and two R's present are the same as or different from each other), and 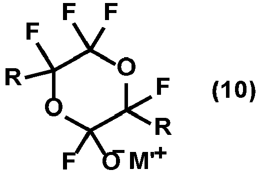 (in Formula (10), M^{'+} represents a counter cation, R represents a fluorine atom or a fluoroalkyl group, and two R's present are the same as or different from each other).

2. The manufacturing method of a 1,4-dioxane derivative according to claim 1, wherein the reaction is carried out in the presence of an organic solvent.

3. The manufacturing method of a 1,4-dioxane derivative according to claim 2, wherein the organic solvent is an ether solvent.

4. The manufacturing method of a 1,4-dioxane derivative according to claim 3,
wherein the ether solvent is one or more ether solvents selected from the group consisting of ethylene glycol dimethyl ether, diethylene glycol dimethyl ether, triethylene glycol dimethyl ether, and tetraethylene glycol dimethyl ether.

5. The manufacturing method of a 1,4-dioxane derivative according to claim 1,
wherein the inorganic base is one or more inorganic bases selected from the group consisting of alkali metal hydroxide salts, alkaline earth metal hydroxide salts, alkali metal carbonates, alkaline earth metal carbonates, alkali metal hydrogencarbonates, fluoride salts, and alkaline earth metal hydrogencarbonates.

6. The manufacturing method of a 1,4-dioxane derivative according to claim 5,
wherein the inorganic base is one or more inorganic bases selected from the group consisting of alkali metal carbonates and fluoride salts.

7. The manufacturing method of a 1,4-dioxane derivative according to claim 5,
wherein the inorganic base is an alkali metal carbonate.

8. The manufacturing method of a 1,4-dioxane derivative according to claim 5,
wherein the inorganic base is a fluoride salt.

9. The manufacturing method of a 1,4-dioxane derivative according to claim 1,
wherein a reaction temperature in the reaction is in a range of -20°C to 200°C.

10. The manufacturing method of a 1,4-dioxane derivative according to claim 1,
wherein R is a fluoroalkyl group having 1 to 10 carbon atoms.

11. The manufacturing method of a 1,4-dioxane derivative according to claim 1,
wherein R is a perfluoroalkyl group having 1 to 4 carbon atoms.

12. A manufacturing method comprising:
manufacturing a 1,4-dioxane derivative by the manufacturing method according to any one of claims 1 to 11; and
the manufacturing method of one or more (1,3-dioxolane)-2-carboxylic acid derivatives selected from the group consisting of Formulas (5), (6), (7), and (9) comprising ring reduction of the manufactured 1,4-dioxane derivative under heating:
a (1,3-dioxolane)-2-carboxylic acid fluoride represented by Formula (5): 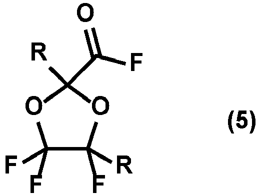 (in Formula (5), R represents a fluorine atom or a fluoroalkyl group, and two R's present are the same as or different from each other),
a (1,3-dioxolane)-2-carboxylic acid represented by Formula (6): 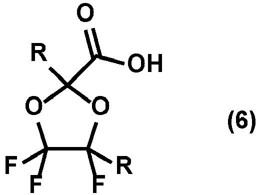 (in Formula (6), R represents a fluorine atom or a fluoroalkyl group, and two R's present are the same as or different from each other),
a (1,3-dioxolane)-2-carboxylic acid salt represented by Formula (7): 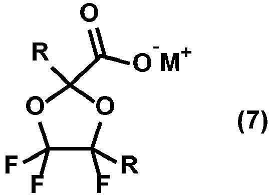 (in Formula (7), M⁺ represents a counter cation, R represents a fluorine atom or a fluoroalkyl group, and two R's present are the same as or different from each other), and
a 1,3-dioxolane derivative represented by Formula (9): 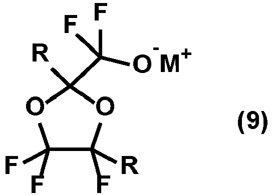 (in Formula (9), M⁺ represents a counter cation, R represents a fluorine atom or a fluoroalkyl group, and two R's present are the same as or different from each other).

13. The manufacturing method of a (1,3-dioxolane)-2-carboxylic acid derivative according to claim 12,
wherein the ring contraction is performed in the presence of a fluoride.

14. The manufacturing method of a (1,3-dioxolane)-2-carboxylic acid derivative according to claim 13,
wherein the fluoride is one or more fluorides selected from the group consisting of cesium fluoride, potassium fluoride, and sodium fluoride.

15. The manufacturing method of a (1,3-dioxolane)-2-carboxylic acid derivative according to claim 12,
wherein a reaction temperature of the ring contraction is in a range of 100°C to 200°C.

16. A 1,4-dioxane derivative represented by Formula (10), 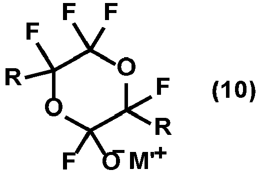 (in Formula (10), M^{'+} represents a counter cation, R represents a fluorine atom or a fluoroalkyl group, and two R's present are the same as or different from each other).

17. A composition comprising:
a 1,4-dioxane derivative represented by Formula (2): 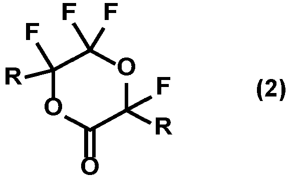 (in Formula (2), R represents a fluorine atom or a fluoroalkyl group in Formula (1), and two R's present are the same as or different from each other); and
a 1,4-dioxane derivative represented by Formula (10): 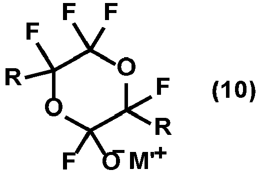 (in Formula (10), M^{'+} represents a counter cation, R represents a fluorine atom or a fluoroalkyl group, and two R's present are the same as or different from each other).

18. A 1,3-dioxolane derivative represented by Formula (9): 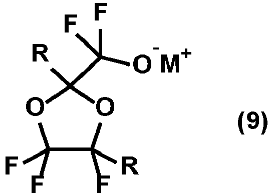 (in Formula (9), M⁺ represents a counter cation, R represents a fluorine atom or a fluoroalkyl group, and two R's are the same as or different from each other).

19. A composition comprising:
one or more (1,3-dioxolane)-2-carboxylic acid derivatives selected from the group consisting of:
a (1,3-dioxolane)-2-carboxylic acid fluoride represented by Formula (5): 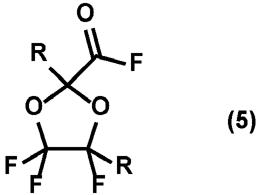 (in Formula (5), R represents a fluorine atom or a fluoroalkyl group, and two R's present are the same as or different from each other),
a (1,3-dioxolane)-2-carboxylic acid represented by Formula (6): 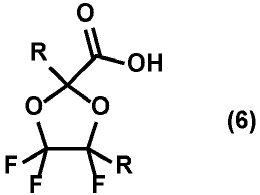 (in Formula (6), R represents a fluorine atom or a fluoroalkyl group, and two R's present are the same as or different from each other), and
a (1,3-dioxolane)-2-carboxylic acid salt represented by Formula (7): 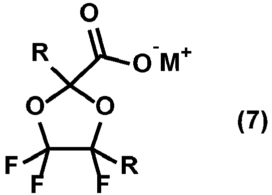 (in Formula (7), M⁺ represents a counter cation, R represents a fluorine atom or a fluoroalkyl group, and two R's present are the same as or different from each other); and
a 1,3-dioxolane derivative represented by Formula (9): 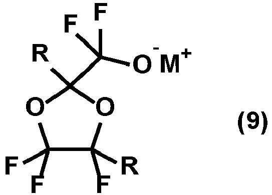 (in Formula (9), M⁺ represents a counter cation, R represents a fluorine atom or a fluoroalkyl group, and two R's present are the same as or different from each other).
